(19) Europäisches Patentamt
European Patent Office
Office européen des brevets

(11) Veröffentlichungsnummer: **0 586 917 A1**

# (12) EUROPÄISCHE PATENTANMELDUNG

(21) Anmeldenummer: **93112920.9**

(22) Anmeldetag: **12.08.93**

(51) Int. Cl.5: **C07C 251/24**, C07D 233/64, C07D 295/30, A61K 31/41, A61K 31/535

(30) Priorität: **10.09.92 DE 4230262**

(43) Veröffentlichungstag der Anmeldung: **16.03.94 Patentblatt 94/11**

(84) Benannte Vertragsstaaten: **AT BE CH DE DK ES FR GB GR IE IT LI LU NL PT SE**

(71) Anmelder: **BEHRINGWERKE Aktiengesellschaft**
**Postfach 1140**
**D-35001 Marburg(DE)**

(72) Erfinder: **Matusch, Rudolf**
**Am Hasenküppel 18a**
**D-35041 Marburg(DE)**
Erfinder: **Czech, Jörg**
**Höhenweg 3**
**D-35041 Marburg(DE)**
Erfinder: **Hunz, Manfred**
**Im Gefälle 10**
**D-35039 Marburg(DE)**
Erfinder: **Sedlacek, Hans-Harald**
**Sonnenhang 3**
**3-35041 Marburg(DE)**

## (54) Substituierte Phenole, Verfahren zu ihrer Herstellung und ihre Verwendung zur Behandlung von zellproliferationsbedingten Erkrankungen.

(57) Es werden substituierte Phenole der Formel I

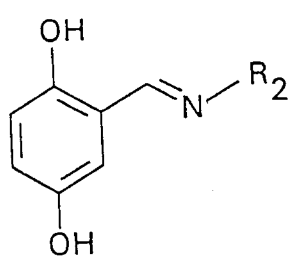

I

Verfahren zu ihrer Herstellung und Mittel enthaltend substituierte Phenole der Formeln I und II

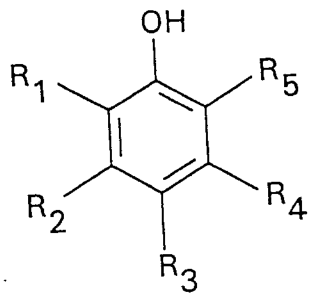

II



Rank Xerox (UK) Business Services
(3.10/3.09/3.3.4)

zur Behandlung von zellproliferationsbedingten Erkrankungen wie Psoriasis, Tumorerkrankungen und immunologische Erkrankungen beschrieben.

Die Erfindung betrifft substituierte Phenole der Formel I, Verfahren zu ihrer Herstellung und Mittel enthaltend substituierte Phenole der Formeln I und II zur Behandlung von zellproliferationsbedingten Erkrankungen wie Psoriasis, Tumorerkrankungen und immunologische Erkrankungen.

Es ist bereits bekannt, daß sich substituierte Phenole zur Behandlung von Tumorerkrankungen eignen (EP 0322738 und EP 0238868), wobei gefunden wurde, daß bestimmte substituierte Phenole die Tyrosinkinaseaktivität des EGF- (epidermal growth factor) Rezeptors inhibieren und damit das Tumorzellwachstum blockieren. Weiter ist bekannt, daß Tyrosinkinasen bei der T- und B-Zellaktivierung des Immunsystems sowie bei der Aktivierung von Mastzellen und basophilen Leukozyten und damit der Histaminausscheidung eine entscheidende Rolle spielen.

Aufgabe der Erfindung ist, bessere Verbindungen zur Behandlung von Tumorerkrankungen und immunologischen Erkrankungen zu erhalten.

Es wurde überraschenderweise gefunden, daß substituierte Phenole der Formeln I und II eine Wirksamkeit gegenüber proliferierenden Tumorzellen aufweisen, wobei sie die EGF-Rezeptortyrosinkinase stark inhibieren.

Die Erfindung betrifft Verbindungen der Formel I

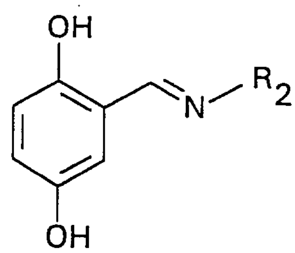

I

wobei

$R_2$ Methyl, Ethyl, Hexyl, Isopropyl, N-Dimethylamino, N-Morpholino, Benzyl, N-Piperidin, N-Homopiperidin, 4-Benzoesäure, 3-Halogenphenyl, 4-Halogenphenyl, N-Piperazin, N-4-Methylpiperazin, oder ein Aminosäurerest mit Ausnahme von Glycin und Methionin, vorzugsweise

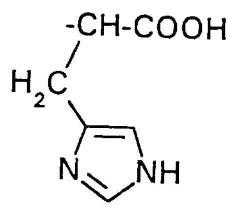

III

oder deren Ester darstellt.

Gegenstand der Erfindung ist auch ein Verfahren zur Herstellung der Verbindungen der Formel I, dadurch gekennzeichnet, daß 2,5-Dihydroxybenzaldehyd in einem geeigneten Lösungsmittel, vorzugsweise Toluol, gelöst oder suspendiert und mit der equimolaren Menge des entsprechenden Amins unter Rückfluß in einer Wasser abscheidenden Apparatur erhitzt wird, bis die Wasserabspaltung beendet ist, oder daß 2,5-Dihydroxybenzaldehyd mit der equimolaren Menge des entsprechenden Amins in getrocknetem Methanol gelöst und auf etwa 60° C erhitzt wird, vorzugsweise etwa 24 h. Nach Abkühlen wird das Lösungsmittel beispielsweise am Rotationsverdampfer unter vermindertem Druck abgezogen. Der Rückstand wird in Toluol/Ethanol aufgenommen und umkristallisiert.

Gegenstand der Erfindung sind außerdem Arzneimittel, die eine Verbindung der Formel I oder eine Verbindung der Formel II

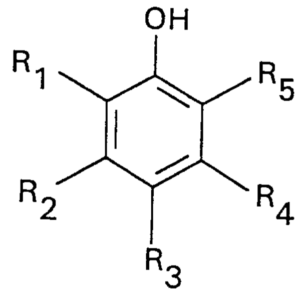

II

enthalten,
wobei

$R_{1,2}$ H oder zusammen ein anelliertes aromatisches System, vorzugsweise ein carbocyclisches System mit vorzugsweise bis zu 8 Kohlenstoffatomen, das substituiert sein kann, vorzugsweise mit bis zu 2 Hydroxygruppen

$R_3$ H oder OH

$R_{4,5}$ unabhängig voneinander H, $C_1$-$C_4$-Alkyl, vorzugsweise Methyl, Sulfonsäure, Aryl, vorzugsweise Phenyl, Hydroxy, CHO oder Halogen, wobei mindestens einer der Reste $R_1$-$R_5$ nicht H ist, oder eine Schiffsche Base der Formel - CH = N - $R_6$ darstellen,

und

$R^6$ Alkyl, N-Dimethylamino, Cyclohexyl, N-Morpholino, Benzyl, N-Piperidin, N-Homopiperidin, Aryl, Halogenaryl, Carboxyaryl oder Hydroxyaryl, wobei Aryl vorzugsweise Phenyl bedeutet, N-Piperazin, N-4-Methylpiperazin oder einen Aminosäurerest, vorzugsweise

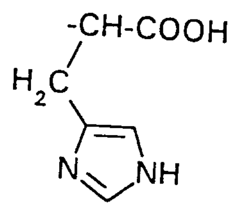

III

oder deren Ester darstellen.

Für $R_3$ = OH betrifft die Erfindung auch die entsprechenden Chinone.

Diese Arzneimittel sind durch einen Gehalt an erfindungsgemäßen substituierten Phenole gekennzeichnet. Die Herstellung der erfindungsgemäßen Arzneimittel erfolgt, indem mindestens ein substituiertes Phenol gegebenenfalls mit weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblichen Hilfsstoffe.

Zur Bekämpfung von Tumorerkrankungen und Immunkrankheiten können die erfindungsgemäßen Verbindungen auf unterschiedliche Weise verabreicht werden. Zum Beispiel können sie intravenös, intramuskulär, intraperitoneal, subkutan, als Dauertropfinfusion, rektal oder oral verabreicht werden. Bei akuten Krankheitszuständen ist die Verabreichung als Dauertropfinfusion vorzuziehen. Zur Dauermedikation ist z.B. die orale Verabreichung angezeigt.

Zum Beispiel können für orale Darreichungsformen Hilfsstoffe wie Stärken, z.B. Kartoffel-, Mais- oder Weizenstärke, Zellulose bzw. deren Derivate, insbesondere mikrokristalline Zellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparates, ein Retardierungsmittel zuzusetzen, gegebenenfalls in Form von permeablen Membranen, wie z.B. solche auf Zellulose- oder Polystyrolharzbasis oder Ionenaustauscher.

Die anzuwendende Dosierung der erfindungsgemäßen Arzneimittel ist abhängig von verschiedenen Faktoren wie Darreichungsform des Medikamentes und Zustand, Gewicht und Art der Erkrankung des

Patienten. Eine Tagesdosis von ca. 5000 mg eines substituierten Phenols sollte jedoch nur kurzzeitig überschritten werden. Bevorzugt sind ca. 10 bis 2500 mg substituiertes Phenol als Tagesdosis für einen Menschen von ca. 70 kg Körpergewicht. Die Verabreichung der Tagesdosis der substituierten Phenole kann in Form einer einzelnen Verabreichung oder in mehreren kleinen Dosen erfolgen. Bevorzugt ist die Verabreichung in 3 bis 8 Dosen pro Tag. In manchen Fällen kann eine kontinuierliche Zuführung der substituierten Phenole z.B. in Form einer Dauertropfinfusion angezeigt sein.

Diese Arzneimittel sind geeignet zur Bekämpfung von Krankheiten, die durch unkontrollierte Zellproliferation (-aktivierung) hervorgerufen werden. Sie sind wirksam zur Inhibition der Zellproliferation, insbesondere der Immun- und Tumorzellproliferation, sowie der Inhibition der Aktivierung von Mastzellen und basophilen Leukozyten, das heißt, zur Behandlung von Psoriasis, Tumorerkrankungen und Immunkrankheiten.

**Beispiele**

**Beispiele 1 - 14:**

**Allgemeine Arbeitsvorschrift zur Darstellung von N-substituierten 2,5-Dihydroxybenzaldiminen (Methode A)**

250 mg (1.8 mmol) 2,5-Dihydroxybenzaldehyd werden mit der equimolaren Menge des entsprechenden Amins in Toluol gelöst oder suspendiert und 12 h unter Rückfluß in einer Wasser abscheidenden Apparatur erhitzt. Nach Abkühlen wird das Lösungsmittel am Rotationsverdampfer unter vermindertem Druck abgezogen. Der Rückstand wird in Toluol/Ethanol aufgenommen und umkristallisiert.

**Allgemeine Arbeitsvorschrift zur Darstellung von N-substituierten 2,5-Dihydroxybenzaldiminen (Methode B)**

250 mg (1.8 mmol) 2,5-Dihydroxybenzaldehyd werden mit der equimolaren Menge des entsprechenden Amins in getrocknetem Methanol gelöst und 24 h auf 60 °C erhitzt. Nach Abkühlen wird das Lösungsmittel am Rotationsverdampfer unter vermindertem Druck abgezogen. Der Rückstand wird in Toluol/Ethanol aufgenommen und umkristallisiert.

| | Amin | Methode | Fp. (°C) | Ausbeute (% d. Th.) |
|---|---|---|---|---|
| 1. 4-[N-(2',5'-Dihydroxybenzyliden)-amino]benzoesäure | 4-Aminobenzosäure | A | 205 | 78 |
| 2. 2,5-Dihydroxy-N-methyl-benzaldimin | Methylamin | B | 122 | 65 |
| 3. N-Benzyl-2,5-dihydroxy-benzaldimin | Benzylamin | A | 118 | 40 |
| 4. 2,5-Dihydroxy-N,N-dimethyl-benzaldehydhydrazon | N,N-Dimethyl-hydrazin | B | 111 | 25 |
| 5. (S)-α-N-(2,5-Dihydroxybenzy-liden)-histidinmethylester | Histidinmethyl-ester | B | 110 | 15 |
| 6. N-Hexyl-2,5-dihydroxy-benzaldimin | Hexylamin | A | 95 | 75 |
| 7. N-Ethyl-2,5-dihydroxy-benzaldimin | Ethylamin | B | 145 | 75 |
| 8. 2,5-Dihydroxy-N-isopropyl-benzaldimin | Isopropylamin | B | 135 | 70 |

| | Amin | Methode | Fp. (°C) | Ausbeute (% d. Th.) |
|---|---|---|---|---|
| 9. N-(4′-Chlorphenyl)-2,5-dihydroxybenzaldimin | 1-Amino-4-chlor-benzol | A | 170 | 70 |
| 10. 2,5-Dihydroxy-N-piperidino-benzaldimin | 1-Aminopiperidin | B | 177 | 50 |
| 11. 2,5-Dihydroxy-N-morpholino-benzaldimin | 4-Aminomorpholin | B | 172 | 60 |
| 12. 2,5-Dihydroxy-N-(4′-methyl-piperazino)benzaldimin | 1-Amino-4-methyl-piperazin | B | 250 | 65 |
| 13. (S)-N-(2′,5′-Dihydroxyben-zyliden)tyrosinmethylester | Tyrosin | B | 131 | 20 |
| 14. (S)-N-(2′,5′-Dihydroxyben-zyliden)-phenylalanin-methylester | Phenylalanin | B | 107 | 20 |

**Beispiel 15:**

**Test auf EGF-Rezeptortyrosinkinasehemmung**

Ausgangsmaterial für die Tyrosinkinaseaktivität war die Human-Tumorzellinie A 431 (ATCC CRL 1555), die in RPMI 1640 Medium + 10 % FKS kultiviert wurde. Diese Zellinie exprimiert auf der Zelloberfläche eine große Zahl EGF-Rezeptoren, welche Tyrosinkinaseaktivität besitzen. Die Zellen wurden bis fast zur Konfluenz kultiviert, mit PBS (Phosphate Buffered Saline, pH 7.2) gewaschen, von der Kulturflasche abgeschabt und 1 h bei 4° C inkubiert, 20x gepottert und 30' bei 1000 g zentrifugiert. Der Überstand wurde weitere 20 min bei 20000 g zentrifugiert und das Pellet als Membranpräparation pro 1 x $10^6$ Zellen in 100 µl aufgenommen.

Die Tyrosinkinaseaktivität des EGF-Rezeptors (EGFRTK) wurde mit Poly (Glu, Ala, Tyr), 6:3:1, als Substrat gemessen. Die Zellmembranen wurden 15' bei RT mit 1000 nM EGF vorbehandelt und dann zu dem Ansatz, der den Inhibitor, Substrat (3 mg/ml), $Mg^{2+}/Mn^{2+}$ (8 mM/1,6 mM), 0.16 % Triton X-100 und Natriumorthovanadat (20 µM) in 100 mM HEPES (N- 2-Hydroxyethyl-Piperazin-N'-2-Ethansulfonsäure), pH 7.5, enthält, gegeben, vorinkubiert und die Reaktion durch Zugabe von gamma-$^{32}P$ ATP (32 µM) begonnen. Nach 15' bei 30° C wurde das Substrat mit 10 % TCA (Trichloressigsäure) gefällt, auf eine Millititer Filtration Plate (Millipore Corporation, Mass., USA) filtriert, gewaschen und getrocknet. Der Einbau von $^{32}P$ wurde mittels eines Flüssigkeitsszintillationszählers bestimmt.

Ergebnisse:

Die substituierten Phenole wurden bei einer Maximalkonzentration von 51 µg/ml geprüft und schrittweise 1:10 verdünnt. $IC_{50}$ bezeichnet die Konzentration, bei der 50 % der Ausgangsenzymaktivität gehemmt wurde (Tabelle 1).

**Beispiel 16:**

**Test auf 3',5'-cAMP-abhängige Proteinkinasehemmung**

Die katalytische Untereinheit der cAMP-abhängigen Proteinkinase (PKA) (Sigma) wurde, wie von Sigma (Sigma Chemical Co., St. Louis, MO, USA) beschrieben, rekonstituiert. Die Enzymaktivität wurde mit Kemptid (Sigma) (Leu-Arg-Arg-Ala-Ser-Leu-Gly) als Substrat gemessen. Der Inhibitor wurde mit Enzym, Substrat (190 µM), $Mg^{2+}$ (5 mM), 0.25 mg/ml BSA und 3.75 mM Mercaptoethanol in 50 mM MOPS (4-Morpholinopropansulfonsäure), pH 6.9, vorinkubiert. Die Reaktion wurde durch Zugabe von gamma-$^{32}P$ ATP (40 µM) begonnen. Nach 15' bei 30° C wurde ein aliquoter Teil auf p81-Ionenaustauscher (2 x 2 cm; Whatman Paper Ltd, Großbritannien) gegeben, in 75 mM $H_3PO_4$ getaucht, gewaschen, getrocknet, und der Einbau von $^{32}P$ wurde mittels eines Flüssigkeitsszintillationszählers bestimmt.

Ergebnisse:

Die substituierten Phenole wurden bei einer Maximalkonzentration von 40 µg/ml geprüft, und die %-Inhibition gibt die Hemmung der Ausgangsenzymaktivität an (Tabelle 1).

Tabelle 1

**Hemmung der enzymatischen Aktivität von Proteinkinasen durch substituierte Phenole.**

| **Substanz** | **EGFRTK** | **PKA** |
|---|---|---|
| | $IC_{50}$, µg/ml | %-Inhib. at 40 µg/ml |
| Hydrochinon | 0.1 | n.t. |
| Benzochinon | 0.05 | 45 |
| Menadiol | 26.2 | 0 |
| Naphthochinon | 0.2 | 46 |
| 2-Methylhydrochinon | 1.1 | 46 |
| Hydrochinon-2-sulfonsäurekaliumsalz | 1.84 | 1 |
| 2-Phenylhydrochinon | 1.03 | 25 |
| 2-tert. Butylhydrochinon | 0.95 | 22 |
| Brenzcatechin | 1.6 | 0 |
| Gentisinaldehyd | 20-30 | n.t. |
| 1,2,4-Trihydroxybenzol | 24.0 | 0 |
| 1,4-Naphthodiol | 1.5 | 76 |
| 1,4-Anthrachinon | 0.58 | n.t. |
| 2,5-Dihydroxy-N-(4'-hydroxyphenyl)benzaldimin | 12.63 | n.t. |
| 2,5-Dihydroxy-N-phenylbenzaldimin | 19.04 | n.t. |
| 3-[N-(2',5'-Dihydroxybenzyliden)amino]benzoesäure | 20.05 | 0 |
| 4-[N-(2',5'-Dihydroxybenzyliden)amino]benzoesäure (Bsp. 1) | 17.37 | 0 |

| | | |
|---|---|---|
| 5-[N-(2',5'-Dihydroxybenzyliden)amino]-2-hydroxy-benzoesäure | 7.45 | 0 |
| N-Hexyl-2,5-dihydroxybenzaldimin (Bsp. 6) | 21.16 | 0 |
| 2,5-Dihydroxy-N-methylbenzaldimin (Bsp. 2) | 1.98 | 0 |
| 2,5-Dihydroxy-N-isopropylbenzaldimin (Bsp. 7) | 12.25 | 0 |
| Chinhydron | 0.126 | 0 |
| N-Isopropyl-2,5-dihydroxybenzaldimin (Bsp. 8) | 8.3 | 0 |
| N-Benzyl-2,5-dihydroxybenzaldimin (Bsp. 3) | 1.7 | 0 |
| 2,3-Dimethylhydrochinon | 0.641 | 71 |
| 2-Chlorhydrochinon | 1.89 | 0 |
| 2-Chlorbenzochinon | 1.38 | 29 |
| 2,5-Dihydroxy-N-piperidinobenzaldimin (Bsp. 10) | 10.51 | 0 |
| 2,5-Dihydroxy-N-morpholinobenzaldimin (Bsp. 11) | 2.77 | 0 |
| 2,5-Dihydroxy-N-(4'-methylpiperazino)benzaldimin (Bsp. 12) | 0.56 | 0 |
| N-Homopiperidino-2,5-dihydroxybenzaldimin | 4.08 | 0 |
| 4-[N-(2',5'-Dihydroxybenzyl)-amino]benzoesäure | 4.61 | 61 |
| N-Cyclohexyl-2,5-dihydroxybenzaldimin | 9.8 | 61 |
| 2,5-Dihydroxy-N,N-dimethylbenzaldehydhydrazon (Bsp. 4) | 1.38 | 63 |
| (S)-alpha-N-(2',5'-Dihydroxybenzyliden)histidinmethylester (Bsp. 5) | 12.64 | 0 |

**Beispiel 17:**

**Test auf Inhibition der EGF bzw. bFGF stimulierten Proliferation von Tumorzellen**

In einer 96-well Mikrotiterplatte wurden 2 x $10^3$ Hela-Zellen pro well ausgesät (in RPMI + 1 % FKS). Nach 24 h wurde das Medium auf RPMI + 0.5 % FKS gewechselt und verschiedene Konzentrationen der Testsubstanz ± EGF (0.1 nM)/bFGF (1 ng/ml) hinzugegeben. Jede Gruppe bestand aus 4 wells, die Kontrolle wurde nur ± Wachstumsfaktor inkubiert. Nach 65 h wurden 50 µl MTT (2.5 mg/ml in PBS) hinzugegeben und nach 7 h der Überstand entfernt. Der von den lebenden Zellen gebildete Farbstoff wurde durch Zugabe von 100 µl DMSO/well gelöst. Die Extinktion wurde für jedes well mit Hilfe eines Multiscan Photometers 340 CC (Fa. Flow) bei 492 nm gemessen.

Ergebnisse:

Aus den 4 wells einer Gruppe wird der Mittelwert gebildet und die Extinktionsdifferenz zwischen den Kontrollen ± Wachstumsfaktor (A) und zwischen den Gruppen mit Testsubstanz ± Wachstumsfaktor (B) bestimmt. Die %-Inhibition der Wachstumsfaktorstimulation errechnet sich nach

$$100 - \frac{B \times 100}{A} \%$$

und ist in Tabelle 2 aufgelistet.

Tabelle 2

| Substanz | EGF-Stimulation % Inhibition bei µg/ml 0.2 | 1 | 5 | 25 |
|---|---|---|---|---|
| Hydrochinon | | 29 | 43 | |
| Benzochinon | | 36 | 55 | |
| Menadiol | | n.t. | | |
| Naphthochinon | 11 | tox | | |
| 2-Methylhydrochinon | | 21 | 66 | |
| Hydrochinon-2-sulfon-säurekaliumsalz | | | 6 | 14 |
| 2-Phenylhydrochinon | | 11 | 66 | |
| 2-tert. Butylhydrochinon | | 21 | 52 | |
| Brenzcatechin | 16 | 35 | | |
| Gentisinaldehyd | | 0 | 16 | |
| 1,2,4-Trihydroxybenzol | | 5 | tox | |
| 1,4-Naphthodiol | 29 | tox | | |
| 1,4-Anthrachinon | | 33 | tox | |
| 2,5-Dihydroxybenzyliden-4-hydroxyanilin | | | 39 | tox |
| 2,5-Dihydroxybenzyliden-anilin | | | 24 | tox |
| (2,5-Dihydroxybenzyliden)-3-aminobenzoesäure | | | 44 | tox |
| (2,5-Dihydroxybenzyliden)-4-aminobenzoesäure (Bsp. 1) | | | 48 | tox |
| (2,5-Dihydroxybenzyliden)-5-amino-2-hydroxy-benzoesäure | | | 51 | tox |
| N-Hexyl-2,5-dihydroxy-benzaldimin (Bsp. 6) | | | 18 | tox |

| | | | |
|---|---|---|---|
| N-Methyl-2,5-dihydroxy-benzaldimin (Bsp. 2) | | 21 | tox |
| N-Ethyl-2,5-dihydroxy-benzaldimin (Bsp. 7) | | 28 | tox |
| Chinhydron | 12 | 64 | |
| N-Isopropyl-2,5-dihydroxy-benzaldimin (Bsp. 8) | | 28 | tox |
| N-Benzyl-2,5-dihydroxy-benzaldimin (Bsp. 3) | | 28 | tox |
| 2,3-Dimethylhydrochinon | | 17 | tox |
| 2-Chlorhydrochinon | | 46 | tox |
| 2-Chlorbenzochinon | | 29 | tox |
| (2,5-Dihydroxybenzyliden)-1-aminopiperidin (Bsp. 10) | | n.t. | |
| (2,5-Dihydroxybenzyliden)-4-aminomorpholin (Bsp. 11) | | 17 | tox |
| (2,5-Dihydroxybenzyliden)-1-amino-4-methylpiperazin (Bsp. 12) | | 21 | tox |
| (2,5-Dihydroxybenzyliden)-1-aminohomopiperidin | | tox | |
| N-2,5-Dihydroxybenzyl-4-aminobenzoesäure | | 36 | tox |
| N-Cyclohexyl-2,5-dihydroxy-benzaldimin | | 39 | 64 |
| N-(2,5-Dihydroxybenzyliden)-N',N'-dimethylhydrazin (Bsp. 4) | | 46 | tox |
| (S)-alpha-N-(2',5'-Dihydroxy-benzyliden)-histidinmethylester (Bsp. 5) | | 10 | 44 |

n.t. = not tested

tox = toxisch

## Patentansprüche

**1.** Verbindungen der Formel I

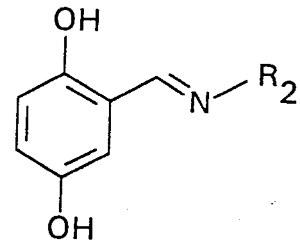

I

wobei

R2 Methyl, Ethyl, Hexyl, Isopropyl, N-Dimethylamino, N-Morpholino, Benzyl, N-Piperidin, N-Homopiperidin, 4-Benzoesäure, 3-Halogenphenyl, 4-Halogenphenyl, N-Piperazin, N-4-Methylpiperazin, oder einen Aminosäurerest mit Ausnahme von Glycin und Methionin, vorzugsweise

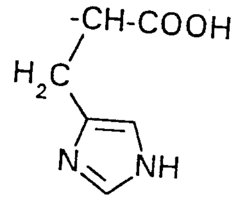

III

oder deren Ester darstellt.

**3.** Arzneimittel, gekennzeichnet durch einen Gehalt an einer Verbindung der Formel I in Anspruch 1 oder der Formel II

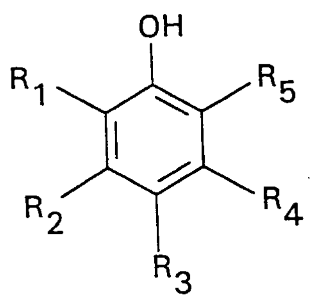

II

wobei

$R_{1,2}$ H oder zusammen ein anelliertes aromatisches System, vorzugsweise ein carbocyclisches System mit vorzugsweise bis zu 8 Kohlenstoffatomen, das substituiert sein kann, vorzugsweise mit bis zu 2 Hydroxygruppen

$R_3$ H oder OH

$R_{4,5}$ unabhängig voneinander H, $C_1$-$C_4$-Alkyl, vorzugsweise Methyl, Sulfonsäure, Aryl, vorzugsweise Phenyl, Hydroxy, CHO oder Halogen, wobei mindestens einer der Reste $R_1$-$R_5$ nicht H ist, oder eine Schiffsche Base der Formel - CH = N - $R_6$ darstellen,

und

$R^6$ Alkyl, N-Dimethylamino, Cyclohexyl, N-Morpholino, Benzyl, N-Piperidin, N-Homopiperidin, Aryl, Halogenaryl, Carboxyaryl oder Hydroxyaryl, wobei Aryl vorzugsweise Phenyl bedeutet, N-Piperazin, N-4-Methylpiperazin, einen Aminosäurerest, vorzugsweise

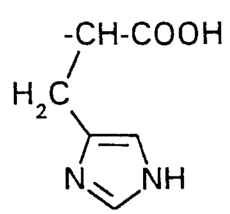

III

oder deren Ester darstellen,
und einen Hilfs- und/oder Trägerstoff.

**3.** Verwendung einer Verbindung der Formel I in Anspruch 1 oder der Formel II in Anspruch 2 zur Herstellung eines Arzneimittels zur Bekämpfung von Krankheiten, die durch unkontrollierte Proliferation von Zellen hervorgerufen werden.

**4.** Verwendung gemäß Anspruch 3 zur Herstellung eines Arzneimittels zur Bekämpfung von Psoriasis, Tumorerkrankungen oder Immunkrankheiten.

**5.** Verfahren zur Herstellung einer Verbindung nach Anspruch I, dadurch gekennzeichnet, daß 2,5-Dihydroxybenzaldehyd in einem Lösungsmittel, vorzugsweise Toluol, gelöst oder suspendiert und mit der equimolaren Menge des entsprechenden Amins unter Rückfluß in einer Wasser abscheidenden Apparatur erhitzt wird, bis die Wasserabspaltung beendet ist, oder daß 2,5-Dihydroxybenzaldehyd mit der equimolaren Menge des entsprechenden Amins in getrocknetem Methanol gelöst und erhitzt wird.

**6.** Verfahren zur Herstellung eines Arzneimittels, dadurch gekennzeichnet, daß eine oder mehrere Verbindung(en) gemäß Formel I in Anspruch 1 oder Formel II in Anspruch 2 mit geeigneten Hilfs- und/oder Trägerstoffen in eine geeignete Darreichungsform gebracht wird.

Europäisches Patentamt

# EUROPÄISCHER RECHERCHENBERICHT

Nummer der Anmeldung
EP 93 11 2920

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | CHEMICAL ABSTRACTS, vol. 110, no. 21, 22. Mai 1989, Columbus, Ohio, US; abstract no. 192427d, H. ULBRICHT ET AL 'Preparation of new azomethines and their derivatives as antiinflammatory agents' Seite 708 ; * Zusammenfassung und RN 120370-64-1 * & DD-A-258 804 (H. ULBRICHT) | 2 | C07C251/24<br>C07D233/64<br>C07D295/30<br>A61K31/41<br>A61K31/535 |
| A | BIOORGANIC & MEDICINAL CHEMISTRY LETTERS Bd. 1, Nr. 4 , 1991 Seiten 215 - 218 M. CUSHMAN ET AL. 'Synthesis and evaluation of new protein-tyrosine kinase inhibitors. Part 2. phenylhydrazones' * abstract; examples, especially ex. 4,18,28 * | 1-6 | |
| A,D | EP-A-0 238 868 (ZAIDANHOJIN BITSEIBUTSU K. K.) * Ansprüche 1,6,7; Beispiele 1,8,18 * | 1-6 | RECHERCHIERTE SACHGEBIETE (Int.Cl.5) |
| A,D | EP-A-0 322 738 (YISSUM RESEARCH & DEVELOPEMENT CO.) * Ansprüche; Beispiele * | 1-6 | C07C<br>C07D |
| A | EP-A-0 183 532 (LILLY INDUSTRIES) * Seite 15, Zeile 24 - Seite 16, Zeile 3; Seite 24, Zeile 18 - Zeile 20; Ansprüche 1,5 * | 1-6 | |

Der vorliegende Recherchenbericht wurde für alle Patentansprüche erstellt

| Recherchenort | Abschlußdatum der Recherche | Prüfer |
|---|---|---|
| DEN HAAG | 10. Dezember 1993 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument